# EUROPEAN PATENT SPECIFICATION

(11) **EP 3 241 543 B1**
(45) Date of publication and mention of the grant of the patent: **29.04.2020**
(21) Application number: 15875673.4
(22) Date of filing: 29.12.2015
(51) Int. Cl.: A61K 8/81, A61K 8/72, A61K 8/89, A61K 8/06, A61K 8/02, A61Q 19/00

(54) **CHEMICALLY ANISOTROPIC POWDER, AND COSMETIC COMPOSITION CONTAINING SAME**
CHEMISCH ANISOTROPES PUDER UND KOSMETISCHE ZUSAMMENSETZUNG DAMIT
POUDRE CHIMIQUEMENT ANISOTROPE, ET COMPOSITION COSMÉTIQUE CONTENANT CELLE-CI

(30) Priority: 31.12.2014 KR 20140194545; 28.12.2015 KR 20150187727
(43) Date of publication of application: 08.11.2017
(73) Proprietor: Amorepacific Corporation, Seoul 140-777 (KR)
(72) Inventor: JIN, Yu Jin, Yongin-si Gyeonggi-do 17074 (KR); NAM, Jin, Yongin-si Gyeonggi-do 17074 (KR); PARK, Hong Guen, Yongin-si Gyeonggi-do 17074 (KR); AN, Soon Ae, Yongin-si Gyeonggi-do 17074 (KR); KANG, Byung Young, Yongin-si Gyeonggi-do 17074 (KR); HAN, Sang Hoon, Yongin-si Gyeonggi-do 17074 (KR)
(74) Representative: Agasse, Stéphane
(86) International application number: PCT/KR2015/014410
(87) International publication number: WO 2016/108579

(56) References cited:
- WO-A1-2015/183042
- KR-A- 20090 073 368
- KR-A- 20140 073 211
- KR-A- 20140 091 556
- KR-B1- 101 299 148
- US-A1- 2006 204 469
- BAO LIU ET AL: "Robust Anisotropic Composite Particles with Tunable Janus Balance", MACROMOLECULES, vol. 45, no. 12, 26 June 2012 (2012-06-26) , pages 5176-5184, XP55250075, US ISSN: 0024-9297, DOI: 10.1021/ma300409r
- LI CHENG ET AL: "A facile fabrication of amphiphilic Janus and hollow latex particles by controlling multistage emulsion polymerization", JOURNAL OF COLLOID AND INTERFACE SCIENCE, vol. 426, 3 April 2014 (2014-04-03), pages 39-43, XP029029675, ISSN: 0021-9797, DOI: 10.1016/J.JCIS.2014.03.061
- TAKUYA TANAKA ET AL: "Dual Stimuli-Responsive "Mushroom-like" Janus Polymer Particles as Particulate Surfactants +", LANGMUIR, vol. 26, no. 14, 20 July 2010 (2010-07-20) , pages 11732-11736, XP55410378, US ISSN: 0743-7463, DOI: 10.1021/la101237c
- Bao Liu ET AL: "Robust Anisotropic Composite Particles with Tunable Janus Balance", MACROMOLECULES, vol. 45, no. 12, 26 June 2012 (2012-06-26) , pages 5176-5184, XP055250075, WASHINGTON, DC, UNITED STATES ISSN: 0024-9297, DOI: 10.1021/ma300409r

## Description

### [Technical Field]

The present disclosure relates to chemically anisotropic powder having improved emulsion dispersion stability and a cosmetic composition including the same.

### [Background Art]

It is known that an emulsion system using a conventional surfactant may be produced stably without demulsification phenomena, such as creaming, flocculation, coalescence or breaking, only when it is produced to have a uniform particle size as small as several micrometers. Therefore, many studies have been conducted to date while being focused on production of an emulsion formulation including emulsion particles having a uniform size as small as several micrometers or less.

However, production of such an emulsion formulation requires stabilization of the interface film using an excessive amount of surfactant as compared to oil content and a decrease in flowability of emulsion particles through an increase in viscosity using a thickener or wax. This results in many problems, such as an unpleasant sticky and stiff feeling of use or limitation in viscosity or soil formation caused by the use of a thickener, thereby making it difficult to provide various types of formulations.

Meanwhile, various methods for preparing fine particles (nano-size, micro-size, etc.) having different shapes and sizes have been reported. Particularly, spherical microparticles including polymers have a size and shape controllable according to preparation methods thereof, and thus have high applicability. For example, there is provided a Pickering emulsion which uses spherical microparticles to form stabilized macroemulsion particles. In addition, some attempts have been made to impart amphiphilic property (i.e. both hydrophilic property and hydrophobic property) to spherical microparticles so that novel anisotropic powder may be obtained. This may be exemplified by Janus spherical particles. However, such spherical particles have a limitation in chemical anisotropy due to their morphological limitation. In other words, although the particles are morphologically anisotropic, they may be hydrophobic or hydrophilic as a whole, and thus have limited chemical anisotropy. Further, due to a difference in specific gravity caused by the size of emulsion particles, phase separation (creaming of an upper layer in the case of an O/W formulation, and precipitation of a lower layer in the case of a W/O formulation) may occur.

Under these circumstances, it is required to provide anisotropic powder capable of increasing dispersibility of macroemulsion particles even with a small amount of thickener (e.g. carbomer) to prevent phase separation, and a cosmetic composition including the same.

### [Disclosure]

### [Technical Problem]

A technical problem to be solved by the present disclosure is to provide chemically anisotropic powder which uses anisotropic powder to increase electrostatic repulsion force so that an increase in external viscosity caused by the use of a thickener and wax may be minimized and a dispersion state may be maintained.

Another technical problem to be solved by the present disclosure is to provide a cosmetic composition which is a stable formulation including macroemulsion particles, causes no coalescence even when the emulsion particles have a non-uniform size, and shows improved dispersion stability among particles to minimize the use of an external thickener.

Still another technical problem to be solved by the present disclosure is to provide a low-viscosity emulsion cosmetic composition having a high oil content which shows a lotion-like softening effect and moisturizing effect, and has a low-viscosity or non-viscous emulsion formulation like skin softener or toner to provide excellent spreadability and feeling of use.

### [Technical Solution]

In one general aspect, there is provided chemically anisotropic powder having improved dispersibility which includes a first polymer spheroid and a second polymer spheroid, wherein the first and second polymer spheroids are bound to each other with a structure in which one polymer spheroid at least partially infiltrates the other polymer spheroid; the first polymer spheroid has a core-shell structure; the shell has a functional group; and at least one of the first polymer spheroid and the second polymer spheroid is negatively or positively charged, wherein the second polymer spheroid and the core of the first polymer spheroid include a vinyl polymer, and the shell of the first polymer spheroid includes a copolymer of a vinyl monomer with a functional group which is siloxane, and the vinyl polymer includes polystyrene.

According to an embodiment, at least one of the first polymer spheroid and the second polymer spheroid may include an ionic vinyl polymer.

According to another embodiment, the ionic vinyl polymer may be a sodium 4-vinylbenzene sulfonate polymer.

According to still another embodiment, the chemically anisotropic powder may have a symmetric shape, asymmetric snowman shape or asymmetric reverse snowman shape on the basis of the binding portion where the first polymer spheroid and the second polymer spheroid are bound to each other.

According to yet another embodiment, the chemically anisotropic powder may have a particle size of 100-1500 nm.

In another general aspect, there is provided a cosmetic composition including chemically anisotropic powder and having improved dispersibility.

According to an embodiment, the chemically anisotropic powder may form macroemulsion particles having a size of 5-200

According to another embodiment, the chemically anisotropic powder may be present in an amount of 0.1-15 wt% based on the total weight of the cosmetic composition.

According to still another embodiment, the cosmetic composition may have an oil content of 15-30 wt% based on the total weight of the cosmetic composition.

According to yet another embodiment, the cosmetic composition may have an emulsion formulation having a low viscosity of 300 cps or less.

### [Advantageous Effects]

According to the embodiments of the present disclosure, the surface of anisotropic powder is modified to minimize the amount of a thickener used for dispersing emulsion particles, thereby providing an emulsion system that allows homogeneous dispersion through the repulsion force between powdery emulsion particles without any thickener. Such an emulsion system prevents phase separation even with a minimized amount of thickener and provides a skin softener-like low-viscosity formulation having a high oil content, which, otherwise, cannot be accomplished according to the related art.

In addition, according to the embodiments of the present disclosure, there is provided a cosmetic composition which includes chemically anisotropic powder having improved dispersibility. Thus, the cosmetic composition is a stable formulation including macroemulsion particles, causes no coalescence even when the emulsion particles have a non-uniform size, and shows improved dispersion stability among particles to minimize the use of an external thickener.

Further, according to the embodiments of the present disclosure, there is provided a low-viscosity emulsion cosmetic composition having a high oil content which shows a lotion-like softening effect and moisturizing effect, and has a low-viscosity or non-viscous emulsion formulation like skin softener or toner to provide excellent spreadability and feeling of use.

### [Description of Drawings]

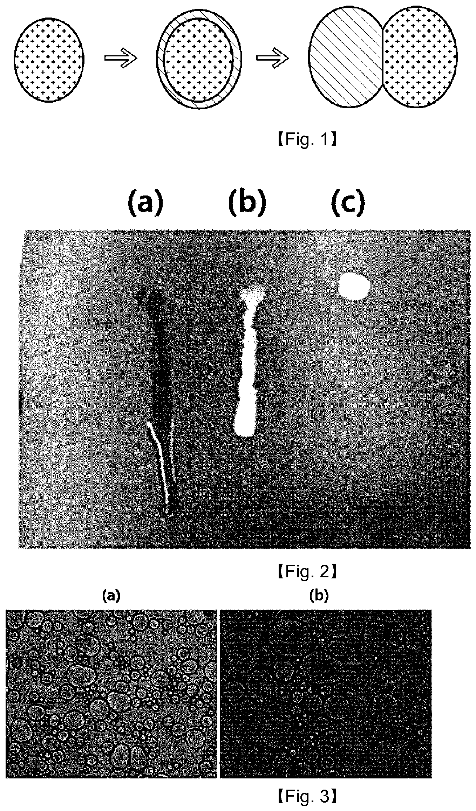

Fig. 1 shows a schematic view illustrating formation of chemically anisotropic powder.
Fig. 2 is an image showing the results of viscosity measurement in the low-viscosity emulsion cosmetic composition having a high oil content disclosed herein (when viewed from the left side, (a) shows a conventional skin softener formulation, (b) shows the formulation of Preparation Example 1, and (c) shows a conventional lotion formulation).
Fig. 3 shows the images the low-viscosity emulsion cosmetic composition having a high oil content disclosed herein as observed by a microscope (the left image (a) is taken at a magnification of x200 and the right image (b) is taken at a magnification of x400, which demonstrates that the chemically anisotropic powder disclosed herein forms macroemulsion particles having a size of several tens of micrometers ranging from at least 15 to 60 µm).

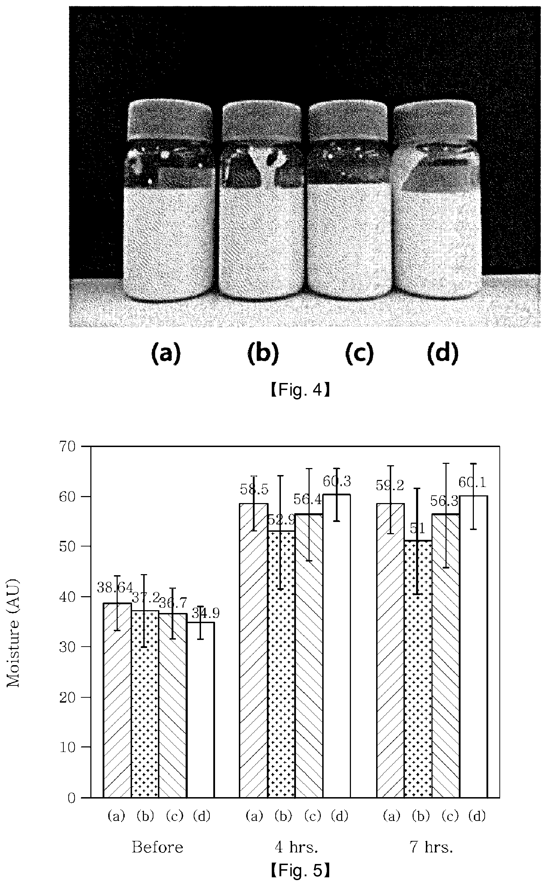

Fig. 4 shows the results of evaluation for formulation stability of the emulsion cosmetic composition disclosed herein (when viewed from the left side, (a) shows the sample stored at room temperature, (b) shows the sample stored at 45°C, (c) shows the sample stored at 60°C, and (d) shows the sample stored under cooling for 4 weeks).
Fig. 5 shows the results of the moisturizing effect of (a) the low-viscosity emulsion cosmetic composition having a high oil content (Preparation Example 1) as compared to the moisturizing effect of each of (b) a conventional skin softener, (c) conventional lotion, and (d) conventional cream (when viewed from the left side, the graph bars represent (a) the cosmetic composition of Preparation Example 1, (b) a conventional skin softener, (c) conventional lotion and (d) conventional cream).

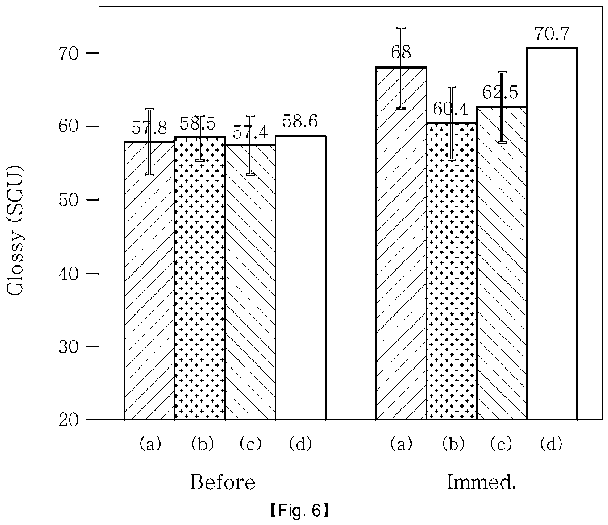

Fig. 6 shows the results of the skin gloss of (a) the low-viscosity emulsion cosmetic composition having a high oil content (Preparation Example 1) as compared to the moisturizing effect of each of (b) a conventional skin softener, (c) conventional skin, and (d) conventional cream (when viewed from the left side, the graph bars represent (a) the cosmetic composition of Preparation Example 1, (b) a conventional skin softener, (c) conventional lotion and (d) conventional cream).

### [Best Mode]

Exemplary embodiments now will be described more fully hereinafter with reference to the accompanying drawings, in which exemplary embodiments are shown. This disclosure may, however, be embodied in many different forms and should not be construed as limited to the exemplary embodiments set forth therein. Rather, these embodiments are provided so that this disclosure will be thorough and complete, and will fully convey the scope of the present disclosure to those skilled in the art. In the drawings, the shape, size and regions, and the like, of the drawing may be exaggerated for clarity. In addition, although a part of constitutional elements is shown for convenience of description, the remaining part may be understood with ease by those skilled in the art. Further, it will be understood by those skilled in the art that various changes in form and details may be made thereto without departing from the scope of this disclosure as defined by the appended claims.

As used herein, "substituted" means that at least one hydrogen atom of the functional group described herein is substituted with a halogen atom (F, CI, Br or I), hydroxyl group, nitro group, imino group (=NH, =NR, wherein R is a C1-C10 alkyl group), amidino group, hydrazine or hydrazine group, carboxyl group, substituted or non-substituted C1-C20 alkyl group, substituted or non-substituted C3-C30 heteroaryl group, or substituted or non-substituted C2-C30 heterocycloalkyl group, unless otherwise stated.

As used herein, "(meth)acryl" means acryl and/or methacryl.

As used herein, the particle size of amphiphilic anisotropic powder is measured as the maximum length that is the largest length of the powder particles. As used herein, the particle size range of amphiphilic anisotropic powder means that at least 95% of the amphiphilic anisotropic powder present in a composition belongs to the corresponding range.

As used herein, the average particle diameter of emulsion particles means the average of diameter of each particle. As used herein, the average particle diameter range of emulsion particles means that at least 95% of the emulsion particles present in a composition belongs to the corresponding range.

In one aspect, there is provided chemically anisotropic powder having improved dispersibility which includes a first polymer spheroid and a second polymer spheroid, wherein the first and second polymer spheroids are bound to each other with a structure in which one polymer spheroid at least partially infiltrates the other polymer spheroid; the first polymer spheroid has a core-shell structure; the shell has a functional group; and at least one of the first polymer spheroid and the second polymer spheroid is negatively or positively charged, wherein the second polymer spheroid and the core of the first polymer spheroid include a vinyl polymer, and the shell of the first polymer spheroid includes a copolymer of a vinyl polymer with a functional group which is siloxane, and wherein the vinyl polymer includes polystyrene.

As used herein, a spheroid means a single body formed of polymers. For example, it may have a spherical or ellipsoidal shape and a micro-scale or nanoscale long axis length based on the largest length in the section of the body.

According to an embodiment, at least one of the first polymer spheroid and the second polymer spheroid may include an ionic vinyl polymer. The ionic vinyl polymer imparts positive charges or negative charges to the chemically anisotropic powder and prevents coalescence (binding) of particles electrostatically. In this manner, it is possible to minimize the use of a thickener when applying the chemically anisotropic powder to a composition and to prevent coalescence of particles, thereby providing a stable emulsion composition.

According to another embodiment, the ionic vinyl polymer may be a sodium 4-vinylbenzene sulfonate.

According to still another embodiment, the amphiphilic anisotropic powder may have a symmetric shape, asymmetric snowman shape or asymmetric reverse snowman shape on the basis of the binding portion where the first polymer spheroid and the second polymer spheroid are bound to each other.

According to yet another embodiment, the amphiphilic anisotropic powder may have a particle size of 100-1500 nm. In a variant, the amphiphilic anisotropic powder may have a particle size of 100-500 nm, or 200-300 nm. Herein, the particle size means the largest length of the amphiphilic powder. Particularly, the amphiphilic powder may have a particle size of at least 100 nm, at least 200 nm, at least 300 nm, at least 400 nm, at least 500 nm, at least 600 nm, at least 700 nm, at least 800 nm, at least 900 nm, at least 1000 nm, at least 1100 nm, at least 1200 nm, at least 1300 nm or at least 1400 nm, and at most 1500 nm, at most 1400 nm, at most 1300 nm, at most 1200 nm, at most 1100 nm, at most 1000 nm, at most 900 nm, at most 800 nm, at most 700 nm, at most 600 nm, at most 500 nm, at most 400 nm, at most 300 nm or at most 200 nm.

In another general aspect, there is provided a cosmetic composition including chemically anisotropic powder and having improved dispersibility.

According to an embodiment, the chemically anisotropic powder may form macroemulsion particles having a size of 5-200 µm. In a variant, the chemically anisotropic powder may form macroemulsion particles having a size of 10-100 µm, 10-50 µm, or 25 µm. Particularly, the chemically anisotropic powder may form emulsion particles having a size of at least 5 µm, at least 10 µm, at least 15 µm, at least 20 µm, at least 25 µm, at least 30 µm, at least 40 µm, at least 50 µm, at least 80 µm, at least 100 µm, at least 130 µm, at least 150 µm or at least 180 µm, and at most 200 µm, at most 180 µm, at most 150 µm, at most 130 µm, at most 100 µm, at most 80 µm, at most 50 µm, at most 40 µm, at most 30 µm, at most 25 µm, at most 20 µm, at most 15 µm or at most 10 µm.

The chemically anisotropic powder has different orientability to the interface so that stable macroemulsion particles may be formed. In addition, the cosmetic composition including the chemically anisotropic powder provides unique double feelings of use, and thus may show a moisturizing effect derived from the watering of macroemulsion particles and then a nourishing effect derived from the applicability of the smaller emulsion particles. In addition, since the repulsion force between the particles of the chemically anisotropic powder is increased, the macroemulsion particles are dispersed with minimized viscosity so that an emulsion of a low-viscosity O/W formulation may be formed.

It is difficult to form stabilized macroemulsion particles having a particle diameter of several tens of micrometers, when using a conventional molecular level surfactant. In this case, such a surfactant forms an interface film having a thickness of merely about several nanometers. However, when using the chemically anisotropic powder disclosed herein, the thickness of an interface film is increased to about several hundreds of nanometers and a stabilized interface film is formed by virtue of the strong binding between powder particles. In this manner, it is possible to improve emulsion stability.

According to an embodiment, the chemically anisotropic powder may be present in an amount of 0.1-15 wt% based on the total weight of the cosmetic composition. In a variant, the chemically anisotropic powder may be present in an amount of 0.5-5 wt% based on the total weight of the cosmetic composition. Particularly, the chemically anisotropic powder may be present in an amount of at least 0.1 wt%, at least 0.5 wt%, at least 1 wt%, at least 2 wt%, at least 4 wt%, at least 6 wt%, at least 8 wt%, at least 10 wt% or at least 12 wt%, and at most 15 wt%, at most 12 wt%, at most 10 wt%, at most 8 wt%, at most 6 wt%, at most 4 wt%, at most 2 wt%, at most 1 wt% or at most 0.5 wt%, based on the total weight of the cosmetic composition. It is possible to control the size of emulsion particles from several micrometers to several tens or several hundreds of micrometers by adjusting the amount of the chemically anisotropic powder.

According to another embodiment, the cosmetic composition may be an emulsion composition. Particularly, the emulsion may be at least one of oil-in-water (O/W) type, water-in-oil (W/O) type, W/O/W type or O/W/O formulations.

According to still another embodiment, the emulsion may be an oil-in-water (O/W) type formulation including the chemically anisotropic powder, an oil phase part and an aqueous phase part at a weight ratio of 0.1-15:5-60:10-80. In a variant, the emulsion may be an oil-in-water (O/W) type formulation including the chemically anisotropic powder, an oil phase part and an aqueous phase part at a weight ratio of 0.1-5:15-40:50-80. In another variant, the emulsion may be a water-in-oil (W/O) type formulation including the chemically anisotropic powder, an oil phase part and an aqueous phase part at a weight ratio of 1-15:50-80:10-30. The oil phase part may include at least one selected from the group consisting of liquid oil and fat, solid oil and fat, wax, hydrocarbon oil, higher fatty acids, higher alcohols, synthetic ester oil and silicon oil.

According to still another embodiment, the chemically anisotropic powder may be added in combination with the aqueous phase part to provide a cosmetic composition.

According to still another embodiment, the cosmetic composition may include oil in an amount of at least 15 wt%, at least 16 wt%, at least 17 wt%, at least 18 wt%, at least 19 wt% or at least 20 wt%, and at most 30 wt%, at most 29 wt%, at most 28 wt%, at most 27 wt%, at most 26 wt% or at most 25 wt%, based on the total weight of the cosmetic composition. For example, the oil may be present in an amount of 15-30 wt%, 15-25 wt% or 20-25 wt%. The cosmetic composition including oil within the above-defined range not only has stabilized macroemulsion particles but also shows improved particle dispersibility, thereby minimizing the amount of a thickener.

The oil may be ester-based oil, hydrocarbon-based oil, wax, animal oil, vegetable oil, silicon oil or a combination thereof. Particularly, the oil may be at least one selected from the group consisting of: ester-based oil selected from hexyl laurate, dicaprylyl carbonate, diisostearyl malate, butylene glycol dicaprylate/dicaprate, cetyl ethyl hexanoate, triethylhexanoin, dicetearyl dimer dilinoleate, caprylic/capric triglyceride, polyglyceryl-2 triisostearate and pentaerythrytol tetraisostearate; hydrocarbon-based oil selected from polybutene, hydrogenated polyisobutene and phytosqualane; and silicon oil selected from phenyl trimethicone and dimethicone.

According to still another embodiment, the cosmetic composition may have a low-viscosity emulsion formulation that is substantially non-viscous having non-detectable viscosity. As used herein "non-viscous" means low viscosity not detectable by a general viscometer.

In a conventional emulsion system, since emulsion particles have a small size, an excessive amount of surfactant should be added as the oil content increases. In this case, it is also required to use a thickener or a viscosity-increasing material, such as wax, in order to prevent coalescence of emulsion particles and phase separation and to stabilize the emulsion. This results in a negative feeling of use, such as stickiness and stiffness and a lotion-like or cream-like formulation. In this case, it is not possible to make a skin softener-like formulation. On the contrary, the chemically anisotropic powder disclosed herein has improved dispersibility, and thus it is possible to make a stable cosmetic emulsion composition having a skin softener-like formulation and a low viscosity of 300 cps or less in the presence of an increased amount of oil up to 25 wt% even with a minimized amount of thickener.

According to still another embodiment, the cosmetic composition may have a viscosity of at most 300 cps. For example, the viscosity may be at most 300 cps, at most 290 cps, at most 280 cps, at most 270 cps, at most 260 cps, at most 250 cps, at most 240 cps, at most 230 cps, at most 220 cps, at most 210 cps, at most 200 cps, at most 190 cps, at most 180 cps, at most 170 cps, at most 160 cps, at most 150 cps, at most 140 cps, at most 130 cps, at most 120 cps, at most 110 cps, at most 100 cps, at most 90 cps, at most 80 cps, at most 70 cps, at most 60 cps, at most 50 cps, at most 40 cps, at most 30 cps or at most 20 cps, and at least 0 cps, at least 1 cps, at least 2 cps, at least 3 cps, at least 4 cps, at least 5 cps, at least 6 cps, at least 7 cps, at least 8 cps, at least 9 cps, at least 10 cps, at least 11 cps, at least 12 cps, at least 13 cps, at least 14 cps, at least 15 cps, at least 16 cps, at least 17 cps, at least 18 cps, at least 19 cps or at least 20 cps. The above viscosity range is for illustrative purposes only and the cosmetic composition may be a non-viscous formulation having non-detectable viscosity.

The cosmetic composition including the chemically anisotropic powder shows increased repulsion force between emulsion particles, and thus can be dispersed under minimized viscosity. Thus, it is possible to provide a low-viscosity O/W type emulsion like a skin softener formulation.

In still another aspect, there is provided a method for preparing chemically anisotropic powder, including: (1) agitating a first monomer which is a styrene monomer and a polymerization initiator to form a core of a first polymer spheroid; (2) agitating the formed core of the first polymer spheroid with a first monomer which is a styrene monomer, a polymerization initiator and a functional group-containing monomer to form a first polymer spheroid having a core-shell structure; and (3) agitating the formed first polymer spheroid having a core-shell structure with a second monomer which is a styrene monomer and a polymerization initiator to obtain anisotropic powder in which a second polymer spheroid is formed.

According to an embodiment, in at least one of step (1) and step (2), an ionic vinyl monomer is mixed and agitated with the polymerization initiator to impart negative charges or positive charges to at least one of the first polymer spheroid and the second polymer spheroid.

In steps (1), (2) and (3), the agitation may be rotary agitation. Since homogeneous mechanical mixing is required together with chemical modification in order to produce uniform particles, rotary agitation is preferred. The rotary agitation may be carried out in a cylindrical reactor but is not limited thereto.

Herein, the internal design of the reactor significantly affects powder formation. The size and position of the baffles of the cylindrical reactor and the distance from an impeller have a significant effect upon the uniformity of the particles to be produced. Preferably, the interval between the internal blade and the blade of an impeller is minimized to make convection flow and intensity thereof uniform, the powdery reaction mixture is introduced to a level lower than the blade length, and the impeller is maintained at a high rotation speed. The rotation speed may be 200 rpm or higher, and the ratio of the diameter to the height of the reactor may be 1-3:1-5. Particularly, the reactor may have a diameter of 10-30 cm and a height of 10-50 cm. The reactor may have a size variable in proportion to the reaction capacity. In addition, the cylindrical reactor may be made of ceramics, glass or the like. The agitation is carried out preferably at a temperature of 50-90°C.

Simple mixing in a cylindrical rotary reactor allows production of uniform particles, requires low energy consumption and provides maximized reaction efficiency, and thus is amenable to mass production. The conventional tumbling method including rotation of a reactor itself causes inclination of the whole part of the reactor with a certain angle and rotation at a high speed, and thus requires high energy consumption and limits the reactor size. Due to the limitation in reactor size, the output is limited to a small amount of approximately several tens of milligrams to several grams. Thus, the conventional tumbling method is not suitable for mass production.

According to an embodiment, the first monomer and the second monomer may be the same or different, and particularly are a styrene monomer. In addition, the first monomer added in step (2) is the same as the first monomer used in step (1) and the initiator used in each step may be the same or different.

The styrene monomer may be substituted or non-substituted styrene, such as at least one selected from the group consisting of styrene, alphamethylstyrene, alpha-ethylstyrene and para-methylstyrene.

According to still another embodiment, the polymerization initiator may be a radical polymerization initiator. Particularly, the polymerization initiator may be at least one selected from peroxide-based and azo-based initiators. In addition, ammonium persulfate, sodium persulfate or potassium persulfate may be used. The peroxide-based radical polymerization initiator may be at least one selected from the group consisting of benzoyl peroxide, lauryl peroxide, cumene hydroperoxide, methylethyl ketone peroxide, t-butyl hydroperoxide, o-chlorobenzoyl peroxide, o-methoxylbenzoyl peroxide, t-butylperoxy-2-ethylhexanoate and t-butylperoxy isobutyrate. The azo-based radical polymerization initiator may be at least one selected from the group consisting of 2,2-azobisisobutyronitrile, 2,2'-azobis(2-methylisobutyronitrile) and 2,2'-azobis(2,4-dimethylvaleronitrile).

According to still another embodiment, in step (1), the first monomer and the polymerization initiator may be mixed at a weight ratio of 100-250:1.

In a variant, in step (1), an ionic vinyl monomer is added together with the first monomer and the polymerization initiator in such a manner that the first monomer, polymerization initiator and the ionic vinyl monomer may be mixed at a weight ratio of 100-250:1:0.001-5. The size and shape of the powder is determined by controlling the size of the first polymer spheroid in step (1), and the size of the first polymer spheroid may be controlled by the ratio of the first monomer, initiator and the ionic vinyl monomer. In addition, it is possible to increase the uniformity of anisotropic powder by mixing the first monomer, polymerization initiator and the ionic vinyl monomer within the above-defined ratio.

According to an embodiment, the ionic vinyl monomer may be sodium 4-vinylbenzene sulfonate. The anisotropic powder obtained by using such an ionic vinyl monomer includes an ionic vinyl polymer, and thus prevents swelling of the particles, and imparts positive or negative charges to the powder surface, thereby preventing coalescence (binding) of the particles electrostatically.

When the chemically anisotropic powder has a size of 200-250 nm, it may be obtained from the first polymer spheroid preparing by reacting the first monomer, initiator and the ionic vinyl monomer at a ratio of 110-130:1:2-4, particularly 115-125:1:2-4, and more particularly 120:1:3.

In addition, when the chemically anisotropic powder has a size of 400-450 nm, it may be obtained from the first polymer spheroid prepared by reacting the first monomer, initiator and the ionic vinyl monomer at a ratio of 225-240:1 :1-3, particularly 230-235:1:1-3, and more particularly 235:1:2.

Further, when the chemically anisotropic powder has a size of 1100-1500 nm, it may be obtained from the first polymer spheroid prepared by reacting the first monomer, initiator and the ionic vinyl monomer at a ratio of 110-130:1:0, particularly 115-125:1:0, and more particularly 120:1:0.

In addition, chemically anisotropic powder having an asymmetric snowman shape may be obtained from the first polymer spheroid prepared by reacting the first monomer, initiator and the ionic vinyl monomer at a ratio of 100-140:1:8-12, particularly 110-130:1:9-11, and more particularly 120:1:10.

Further, chemically anisotropic powder having an asymmetric reverse snowman shape may be obtained from the first polymer spheroid prepared by reacting the first monomer, initiator and the ionic vinyl monomer at a ratio of 100-140:1:1-5, particularly 110-130:1:2-4, and more particularly 120:1:3.

According to still another embodiment, in step (2), the monomer including the first monomer, polymerization initiator and the functional group-containing monomer may be mixed at a weight ratio of 80-98:0.2-0.8:2-20. In a variant, the first monomer, polymerization initiator and the functional group-containing monomer may be mixed at a weight ratio of 160-200:1:6-40. It is possible to control the coating degree according to the reaction ratio, and then the coating degree determines the shape of chemically anisotropic powder. When the first monomer, polymerization initiator and the functional group-containing monomer are used within the above-defined ratio, the coating thickness is increased by about 10-30%, particularly approximately 20%, based on the initial thickness. In this case, formation of powder proceeds smoothly without problems, such as a failure in formation of powder caused by excessively thick coating or multidirectional formation of powder caused by excessively thin coating. In addition, it is possible to increase the uniformity of anisotropic powder within the above weight ratio.

According to still another embodiment, the functional group-containing monomer may be a siloxane-containing compound. Particularly, it may be a siloxane-containing (meth)acrylate monomer, and particularly may be at least one selected from the group consisting of 3-(trimethoxysilyl)propyl acrylate, 3-(trimethoxysilyl)propyl methacrylate, vinyltriethoxysilane and vinyltrimethoxy silane.

According to still another embodiment, in step (3), the second monomer and polymerization initiator may be mixed at a weight ratio of 200-250:1.

In a variant, in step (3), an ionic vinyl monomer may be added together with the second monomer and polymerization initiator in such a manner that the second monomer, polymerization initiator and the ionic vinyl monomer may be mixed at a weight ratio of 200-250:1:0.001-5. Particular examples of the ionic vinyl monomer are the same as described above. It is possible to increase the uniformity of anisotropic powder within the above weight ratio.

According to still another embodiment, in step (3), the second monomer may be mixed in an amount of 40-300 parts by weight based on 100 parts by weight of the first polymer spheroid. Particularly, when the content of the second monomer is 40-100% based on the weight of the first polymer spheroid, asymmetric snowman-like powder is obtained. When the content of the second monomer is 100-150% or 110-150% based on the weight of the first polymer spheroid, symmetric powder is obtained. In addition, when the content of the second monomer is 150-300% or 160-300% based on the weight of the first polymer spheroid, asymmetric reverse snowman-like powder is obtained. It is possible to increase the uniformity of anisotropic powder within the above weight ratio.

According to the related art, many attempts have been made to increase the surface active property of spherical powder particles used for Pickering by imparting amphiphilic surface active property thereto. This may be exemplified by Janus spherical particles. However, such particles are geometrically limited and have a problem in terms of uniform mass production, and thus cannot be applied practically. On the contrary, the method for preparing amphiphilic anisotropic powder disclosed herein uses no crosslinking agent, thereby causing no agglomeration and providing high yield and uniformity. In addition, the method disclosed herein uses a simple agitation process and is more amenable to mass production as compared to a tumbling process. Particularly, the method disclosed herein is advantageous in that it allows production of nano-size particles having a size of 300 nm or less in a large scale of several tens of grams and several tens of kilograms.

### [Mode for Invention]

Exemplary embodiments now will be described more fully hereinafter with reference to the accompanying drawings, in which exemplary embodiments are shown. This disclosure may, however, be embodied in many different forms and should not be construed as limited to the exemplary embodiments set forth therein.

### Preparation Example 1. Preparation of Polystyrene (PS) First Polymer Spheroid

Styrene as a monomer, sodium 4-vinylbenzene sulfonate as an ionic vinyl monomer and azobisisobutyronitrile (AIBN) as an initiator are mixed in an aqueous phase and are allowed to react at 75°C for 8 hours. The reaction is carried out by agitating the reaction mixture in a cylindrical reactor having a diameter of 11 cm and a height of 17 cm and made of glass under a speed of 200 rpm.

### Preparation Example 2. Preparation of Coated First Polymer Spheroid Having Core-Shell (CS) Structure

The polystyrene (PS) first polymer spherical particles obtained as described above are mixed with styrene as a monomer, 3-(trimethoxysilyl)propyl acrylate (TMSPA) and azobisisobutyronitrile (AIBN) as an initiator and the reaction mixture is allowed to react. The reaction is carried out by agitating the reaction mixture in a cylindrical reactor.

### Preparation Example 3. Preparation of Ionic Anisotropic Powder

The aqueous dispersion of the polystyrene-core shell (PC-CS) dispersion obtained as described above is mixed with styrene as a monomer, sodium 4-vinylbenzene sulfonate as an anionic vinyl monomer and azobisisobutyronitrile (AIBN) as an initiator and the reaction mixture is heated to 75°C to carry out reaction. The reaction is carried out by agitating the reaction mixture in a cylindrical reactor.

### Example 1. Low-Viscosity Emulsion Cosmetic Composition Having High Content of Oil

An emulsion cosmetic composition is obtained according to the composition as shown in the following Table 1. The oil phase part is warmed and dissolved and the aqueous phase part containing anisotropic powder is warmed to the same temperature as the oil phase part. Then, the oil phase part is introduced gradually to the aqueous phase part and emulsification is carried out at a low speed. After that, a thickener is introduced and dispersed to obtain a low-viscosity oil-in-water type emulsion cosmetic composition having a high content of oil.

**[Table 1]**

| Ingredients | Amount (wt%) |
|---|---|
| Oil (Hydrogenated Polydecene) | 15.00 |
| Butter (Shea Butter) | 2.00 |
| Water (D. I. water) | 68.55 |
| Metal ion chelator (EDTA 2Na) | 0.05 |
| Moisturizer (Butylene Glycol) | 7.00 |
| Moisturizer (Glycerin) | 5.00 |
| Anisotropic powder | 1.85 |
| Preservative (Phenoxyethanol) | 0.40 |
| Preservative (Ethylhexylglycerin) | 0.05 |
| Perfume | 0.1 |

### Test Example 1. Comparison of Viscosity

Each of the cosmetic composition according to Example 1, a conventional skin softener formulation and a conventional lotion formulation is dropped onto a strawboard sheet in an amount of 0.2 mL and is inclined at an angle of 60°. As a result, it can be seen from Fig. 2 that the cosmetic composition according to Example 1 has high flowability like the conventional skin softener formulation and shows excellent spreadability and feeling of use.

### Test Example 2. Determination of Formulation Stability

The cosmetic composition obtained from Example 1 is stored (a) at room temperature, (b) at 45°C, (c) at 60°C and (d) in a thermostat under cooling. Then, the cosmetic composition is observed for 10 weeks to determine whether demulsification occurs or not. It can be seen from Fig. 4 that the cosmetic composition maintains its formulation stably with no demulsification.

### Test Example 3. Sensational Evaluation for Feeling of Use

Forty female panels in their thirties to forties are allowed to use the cosmetic composition obtained from Example 1. Then, questionnaire survey is carried out to evaluate feelings of use (15 grades, grade 1: very low, grade 10: very high). The cosmetic composition obtained from Example 1 is compared with a conventional skin softener, conventional lotion and conventional cream formulations.

**[Table 2]**

| | Moisturizing effect | Watering | Nourishing effect |
|---|---|---|---|
| Ex. 1 | 8 | 9 | 8 |
| Conventional skin softener | 8 | 8 | 2 |
| Conventional lotion | 5 | 4 | 6 |
| Conventional cream | 4 | 2 | 8 |

As a result, it can be seen that the emulsion particles of the cosmetic composition obtained from Example 1 exist together with no coalescence, and thus the cosmetic composition provides unique double feelings of use, including a moisturizing effect derived from the watering of macroemulsion particles and then a nourishing effect derived from the applicability of the smaller emulsion particles.

### Test Example 4. Evaluation of Moisturizing Effect and Skin Gloss

The cosmetic composition obtained according to Example 1, a conventional skin softener, conventional lotion and conventional cream are applied to the skin of each of 15 in-company research workers as subjects. Then, the moisturizing effect and skin gloss for each sample are determined. Evaluation of moisturizing effect is carried out by measuring the moisture content before the application of each sample, 4 hours after the application and 7 hours after the application under a constant-temperature and constant-humidity (24°C, relative humidity 40%) condition. Evaluation of skin gloss is carried out by measuring the skin gloss before and right after the application of each sample. After cleaning and waiting for 15 minutes under a constant-temperature and constant-humidity condition, the skin gloss is measured at the forearm portion of each subject. The skin moisture content is determined by measuring skin capacitance with a skin moisture content measuring system (Corneometer CM825, C+K Electronic Co., Germany). The results are shown in Fig. 5. The skin gloss is determined by detecting the light reflected regularly after the light with a specific wavelength is emitted from Glossmeter®. The results are shown in Fig. 6.

It can be seen from Fig. 5 and Fig. 6 that the cosmetic composition obtained from Example 1 shows a moisturizing effect and skin gloss like lotion or cream, while it has skin softener-like properties.

(Corneometer CM825, C+K Electronic Co., Germany). The results are shown in Fig. 5. The skin gloss is determined by detecting the light reflected regularly after the light with a specific wavelength is emitted from Glossmeter®. The results are shown in Fig. 6.

It can be seen from Fig. 5 and Fig. 6 that the cosmetic composition obtained from Example 1 shows a moisturizing effect and skin gloss like lotion or cream, while it has skin softener-like properties.

While the exemplary embodiments have been shown and described, it will be understood by those skilled in the art that various changes in form and details may be made thereto without departing from the scope of this disclosure as defined by the appended claims. Therefore, it is intended that the scope of the present disclosure includes all embodiments falling within the spirit and scope of the appended claims.

## Claims

1. Chemically anisotropic powder having improved dispersibility which comprises a first polymer spheroid and a second polymer spheroid,
wherein the first and second polymer spheroids are bound to each other with a structure in which one polymer spheroid at least partially infiltrates the other polymer spheroid;
the first polymer spheroid has a core-shell structure;
the shell has a functional group; and
at least one of the first polymer spheroid and the second polymer spheroid is negatively or positively charged,
wherein the second polymer spheroid and the core of the first polymer spheroid comprise a vinyl polymer, and the shell of the first polymer spheroid comprises a copolymer of a vinyl monomer with a functional group which is siloxane, and
wherein the vinyl polymer comprises polystyrene.

2. The chemically anisotropic powder according to claim 1, wherein at least one of the first polymer spheroid and the second polymer spheroid comprises an ionic vinyl polymer.

3. The chemically anisotropic powder according to claim 2, wherein the ionic vinyl polymer is a sodium 4-vinylbenzene sulfonate polymer.

4. The chemically anisotropic powder according to claim 1, which has a symmetric shape, asymmetric snowman shape or asymmetric reverse snowman shape on the basis of the binding portion where the first polymer spheroid and the second polymer spheroid are bound to each other.

5. The chemically anisotropic powder according to claim 1, which has a particle size of 100-1500 nm.

6. A cosmetic composition comprising the chemically anisotropic powder as defined in any one of claims 1 to 5 and having improved dispersibility.

7. The cosmetic composition according to claim 6, wherein the chemically anisotropic powder forms macroemulsion particles having a size of 5-200 µm.

8. The cosmetic composition according to claim 6, wherein the chemically anisotropic powder is present in an amount of 0.1-15 wt% based on the total weight of the cosmetic composition.

9. The cosmetic composition according to claim 6, which has an oil content of 15-30 wt% based on the total weight of the cosmetic composition.

10. The cosmetic composition according to claim 6, which has an emulsion formulation having a low viscosity of 300 cps or less.

## Patentansprüche

1. Chemisch anisotropes Puder, eine verbesserte Dispergierbarkeit aufweisend, das ein erstes Polymer-Sphäroid und ein zweites Polymer-Sphäroid umfasst,
wobei das erste und zweite Polymer-Sphäroid mit einer Struktur aneinandergebunden sind, in der ein Polymer-Sphäroid das andere Polymer-Sphäroid mindestens teilweise infiltriert;
das erste Polymer-Sphäroid eine Kern-Schale-Struktur aufweist;
die Schale eine funktionelle Gruppe aufweist; und
mindestens eines von dem ersten Polymer-Sphäroid und dem zweiten Polymer-Sphäroid negativ oder positiv geladen ist,
wobei das zweite Polymer-Sphäroid und der Kern des ersten Polymer-Sphäroids ein Vinyl-Polymer umfassen, und die Schale des ersten Polymer-Sphäroids ein Copolymer eines Vinyl-Monomers mit einer funktionellen Gruppe, die Siloxan ist, umfasst, und
wobei das Vinyl-Polymer Polystyrol umfasst.

2. Chemisch anisotropes Puder nach Anspruch 1, wobei mindestens eines von dem ersten Polymer-Sphäroid und dem zweiten Polymer-Sphäroid ein ionisches Vinyl-Polymer umfasst.

3. Chemisch anisotropes Puder nach Anspruch 2, wobei das ionische Vinyl-Polymer ein Natrium-4-Vinylbenzolsulfonat-Polymer ist.

4. Chemisch anisotropes Puder nach Anspruch 1, das eine symmetrische Form, eine asymmetrische Schneemannform oder umgekehrte asymmetrische Schneemannform an der Basis des Bindungsabschnitts aufweist, wo das erste Polymer-Sphäroid und das zweite Polymer-Sphäroid aneinandergebunden sind.

5. Chemisch anisotropes Puder nach Anspruch 1, das eine Teilchengröße von 100-500 nm aufweist.

6. Kosmetische Zusammensetzung, die das chemisch anisotrope Puder nach einem der Ansprüche 1 bis 5 umfasst, und eine verbesserte Dispergierbarkeit aufweist.

7. Kosmetische Zusammensetzung nach Anspruch 6, wobei das chemisch anisotrope Puder Makroemulsionsteilchen bildet, die eine Größe von 5-200 µm aufweisen.

8. Kosmetische Zusammensetzung nach Anspruch 6, wobei das chemisch anisotrope Puder in einer Menge von 0,1-15 Gew.-% basierend auf dem Gesamtgewicht der kosmetischen Zusammensetzung vorhanden ist.

9. Kosmetische Zusammensetzung nach Anspruch 6, die einen Ölgehalt von 15-30 Gew.-% basierend auf dem Gesamtgewicht der kosmetischen Zusammensetzung aufweist.

10. Kosmetische Zusammensetzung nach Anspruch 6, die eine Emulgierformulierung aufweist, die eine niedrige Viskosität von 300 cps oder weniger aufweist.

## Revendications

1. Poudre chimiquement anisotrope présentant une dispersibilité améliorée qui comprend un premier sphéroïde polymère et un deuxième sphéroïde polymère,
dans laquelle les premier et deuxième sphéroïdes polymères sont liés l'un à l'autre avec une structure dans laquelle un sphéroïde polymère s'infiltre au moins partiellement dans l'autre sphéroïde polymère ;
le premier sphéroïde polymère a une structure noyau-enveloppe ;
l'enveloppe a un groupe fonctionnel ; et
au moins l'un du premier sphéroïde polymère et du deuxième sphéroïde polymère est chargé négativement ou positivement,
dans laquelle le deuxième sphéroïde polymère et le noyau du premier sphéroïde polymère comprennent un polymère vinylique, et l'enveloppe du premier sphéroïde polymère comprend un copolymère d'un monomère vinylique avec un groupe fonctionnel qui est le siloxane, et
dans laquelle le polymère vinylique comprend du polystyrène.

2. Poudre chimiquement anisotrope selon la revendication 1, dans laquelle au moins l'un du premier sphéroïde polymère et du deuxième sphéroïde polymère comprend un polymère vinylique ionique.

3. Poudre chimiquement anisotrope selon la revendication 2, dans laquelle le polymère vinylique ionique est un polymère de 4-vinylbenzène sulfonate de sodium.

4. Poudre chimiquement anisotrope selon la revendication 1, qui a une forme symétrique, une forme d'un bonhomme de neige asymétrique ou une forme d'un bonhomme de neige inversé asymétrique sur la base de la partie de liaison où le premier sphéroïde polymère et le deuxième sphéroïde polymère sont liés l'un à l'autre.

5. Poudre chimiquement anisotrope selon la revendication 1, qui a une taille de particule allant de 100 à 1500 nm.

6. Composition cosmétique comprenant la poudre chimiquement anisotrope telle que définie dans l'une quelconque des revendications 1 à 5 et présentant une dispersibilité améliorée.

7. Composition cosmétique selon la revendication 6, dans laquelle la poudre chimiquement anisotrope forme des particules de macroémulsion ayant une taille allant de 5 à 200 µm.

8. Composition cosmétique selon la revendication 6, dans laquelle la poudre chimiquement anisotrope est présente en une quantité allant de 0,1 à 15% en poids par rapport au poids total de la composition cosmétique.

9. Composition cosmétique selon la revendication 6, qui a une teneur en huile allant de 15 à 30% en poids par rapport au poids total de la composition cosmétique.

10. Composition cosmétique selon la revendication 6, qui a une formulation d'émulsion ayant une faible viscosité inférieure ou égale à 300 cps.
